# EUROPEAN PATENT SPECIFICATION

(11) **EP 1 067 118 B1**
(45) Date of publication and mention of the grant of the patent: **31.03.2004**
(21) Application number: 00110015.5
(22) Date of filing: 12.05.2000
(51) Int. Cl.: C07C 255/31, C11B 9/00, A61K 7/46

(54) **Cyclopentylalkyl-nitriles and the use of odoriferous cyclopentylalkyl derivatives as fragrances**
Cyclopentylalkylnitrile und die Verwendung von Cyclopentylalkyl-Derivaten als Duftstoffe
Cyclopentylalkyle-nitriles et l'utilisation de dérivées de cyclopentylalkyle à titre d'ingrédients parfumants

(30) Priority: 05.07.1999 EP 99810585
(43) Date of publication of application: 10.01.2001
(73) Proprietor: Givaudan SA, 1214 Vernier-Genève (CH)
(72) Inventor: Bajgrowicz, Jerzy A., 8053 Zürich (CH); Bourdin Trunz, Bernadette, 1205 Genève (CH); Gygax, Peter, 8117 Fällanden (CH)
(74) Representative: Patentanwälte Schaad, Balass, Menzl & Partner AG

(56) References cited:
- EP-A- 0 694 520
- EP-A- 0 913 383
- DE-A- 2 444 837
- GB-A- 2 053 199
- CH. VON RANSON, H.-D. BELITZ: "Untersuchungen zur Struktur-Aktivitätsbeziehung bei Geruchsstoffen" Z. LEBENSMITTEL-UNTERSUCHUNG UND -FORSCHUNG, vol. 195, no. 6, 1992, pages 523-6, XP000941126
- C. WAWRZEBNCZYK: "Synthesis and Odor Characteristics of Some Cyclopentane Derivatives" PERFUM. FLAVOR., vol. 16, no. 4, 1991, pages 21-3, XP000941125

## Description

The present invention refers to cyclopentylalkylnitriles and to the use of odoriferous cyclopentylalkyl derivatives as fragrances.

Despite the common occurrence of five-membered carbon rings in perfumery ingredients, mainly of terpenic origin (e. g. campholenic aldehyde derivatives of sandalwood-type odor note) or resulting from a Diels-Alder condensation with inexpensive cyclopentadiene, very few of them contain an unsubstituted, isolated (i. e. not making part of a polycyclic fused or spiro system) cyclopentyl, cyclopentenyl or cyclopentylidene radical. The following products figure among the few examples of such perfumery raw materials:

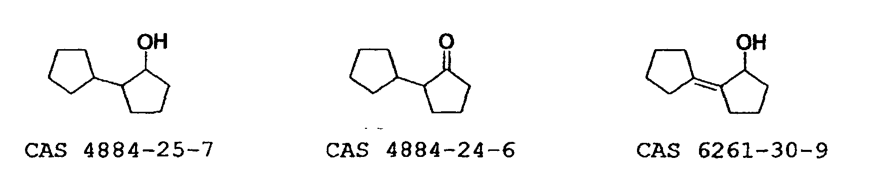

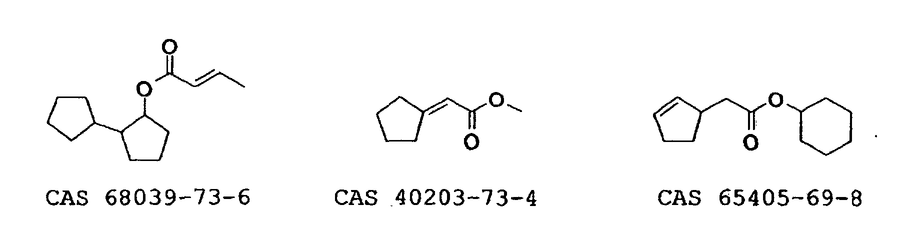

These compounds are also described in EP 0 016 650, EP 0 770 671 and DE 2 729 121.

EP-A-694520 discloses campholenic aldehyde derivatives and their use as perfume chemicals. GB-A-2053199 describes nitriles based on the skeleton of 1,1,2-trimethylcyclopentane and their use for the preparation of perfume compositions. Furthermore, the synthesis and odor characteristics of some cyclopentane derivatives is described (Perfumer and Flavorist, vol. 16, no. 4, 1991, pages 21-23) as well as the threshold values and odor qualities of a number of alicyclic and aromatic aldehydes (Zeitschrift für Lebensmittel-Untersuchung und -Forschung, vol. 195, no. 6, 1992, pages 523-526).

There is a renewed interest in floral fragrances. Therefore, the object of the present invention is to provide perfumery ingredients exhibiting original, intense, diffusive and substantive (i. e. long-lasting) scents belonging to the floral family.

It has been found that new compounds of formula I, 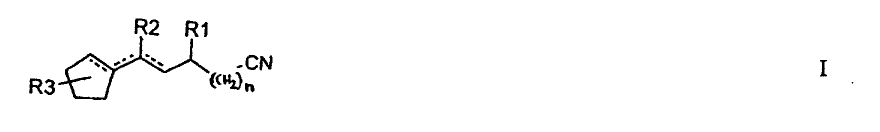 wherein
R¹, R² and R³ are independently H, C₁₋₃ alkyl but R¹ and R² are not at the same time H;
R² can also be methylene or ethylidene;
n = 0 or 1 and
--- stands for a single or a double bond, whereby maximum 2 double bonds are present
possess very intense, mainly rosy and orris notes.

It has further been found that compounds of formula II, 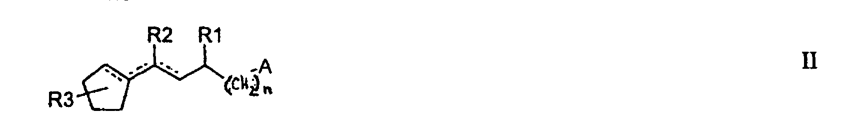 wherein
A is selected from the group of CR⁴R⁵OH, CR⁴R⁵OC(O)R⁶, CO₂R⁶, CN and C(O)R⁴;
R¹, R², R³, R⁴, R⁵ and R⁶ are independently H or C₁₋₃-alkyl;
R² can also be methylene or ethylidene;
R⁶ can also be a C₂₋₄ alkenyl or alkinyl
n = 0 or 1 and
--- stands for a single or a double bond, whereby maximum 2 double bonds are present
possess interesting olfactory properties. The odors are mainly floral, e.g. lily of the valley, orris or ylang-ylang and fruity, e.g. citrus. They are intense, diffusive and long lasting. All compounds of the general formula II have substantive odors, a quality crucial for functional perfumery.

The heteroatoms in all compounds of formula I and II are in a more remote position relative to the lipophilic cyclopentane ring, than the oxygen atoms in the corresponding known perfumery ingredients.

The compounds of formula II may be used to impart odor to any perfumery composition such as fine and functional perfumery, e.g. perfume, fine fragrance accord or detergent, fabric softener, shower gel, soap, cosmetics, scented candle.

The compounds of formula I are new. The following compounds of formula II are also new:
5-Cyclopentyl-3-methylpent-4-en-1-ol
5-Cyclopentyl-3-methylpent-4-enal
5-Cyclopentyl-3-methylpent-4-en-1-yl acetate
5-Cyclopentyl-3-methylpentan-1-ol
4-Cyclopentylpentan-1-ol
4-Cyclopentylpent-1-yl propanoate
4-Cyclopentylpentanal
Ethyl-4-cyclopentylpentanoate
5-Cyclopentylhexan-2-one
5-Cyclopentylhexanal
5-Cyclopentylhexan-1-ol
5-(2-Methylcyclopent-1-enyl)pentan-1-ol
5-(5-Methylcyclopent-1-enyl)pentan-1-ol
4-Cyclopentylidenebutan-1-ol
6-Cyclopentyl-3-methylhexan-3-ol
5-(Cyclopent-1-enyl)-2-methylpentan-2-ol
5-Cyclopentylpentan-2-ol
5-Cyclopentylidene-2-methylpentan-2-ol
5-Cyclopentylidenepent-2-yl propanoate

The following compounds of formula I are preferred:
5-Cyclopentyl-3-methylpentanenitrile
4-Cyclopentylpentanenitrile
5-Cyclopentylhexanenitrile
5-Cyclopentylidenehexanenitrile
5-(Cyclopent-1-enyl)hexanenitrile

The compounds of general formulae I and II can be advantageously prepared by different synthetic ways. Thereby, the five-membered ring is introduced with commercially available starting materials (cyclopentanone, cyclopentadiene, cyclopentylhalogenide and derivatives) or, built e.g. *via* 1,4-dihalogenobutane derived Grignard reagent addition to lactones. Among the best methods of synthesis of functionnalized cyclopentyl-, cyclopentenyl- or cyclopentylidene-alkanes figures the cyclopentadienyl anion addition to carbonyl group, followed by a total or partial hydrogenation of the thus formed fulvene structure, as depicted in scheme I (e. g. Coe, J.; Vetelino, M. G.; Kemp, D. S. *Tetrahedron Lett.* **1994**, *35*, 6627.).

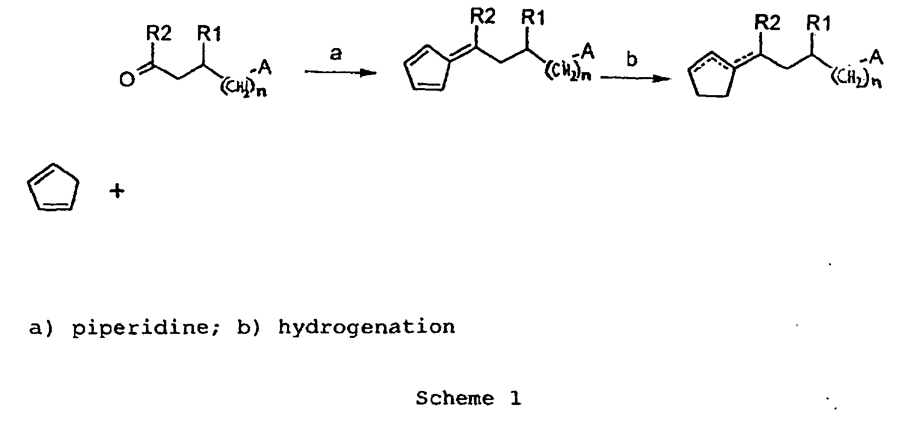

The odorants of formula II may be combined with numerous odorant ingredients of natural and/or synthetic origin, whereby the range of the natural odorants can include not only readily volatile, but also moderately and only slightly volatile components, and the synthetic ones can embrace representatives from practically all classes of substances. The following list comprises examples of known odorants which may be combined with the compounds of the invention:
natural products: such as tree moss absolute, basil oil, tropical fruit oils (such as bergamot oil, mandarine oil), mastix absolute, myrtle oil, palmarosa oil, galbanum oil, patchouli oil, petitgrain oil, wormwood oil, lavender oil, rose oil, jasmine oil, ylang-ylang oil;
alcohols: such as farnesol, geraniol, linalool, nerol, phenylethyl alcohol, rhodinol, cinnamic alcohol, (Z)-hex-3-en-1-ol, menthol, α-terpineol;
-aldehydes: such as citral, α-hexyl cinnamaldehyde, Lilial® (Givaudan Roure), hydroxycitronellal, methylnonylacet-aldehyde, phenylacetaldehyde, anisaldehyde, vanillin;
ketones: such as allylionones, α-ionone, β-ionone, Isoraldeine® (Givaudan Roure), methylionone, verbenone, nootkatone, geranylacetone;
esters: such as allyl phenoxyacetate, benzyl salicylate, cinnamyl propionate, citronellyl acetate, decyl acetate, dimethylbenzylcarbinyl acetate, dimethylbenzylcarbinyl butyrate, ethyl acetoacetate, cis-3-hexenyl isobutyrate, cis-3-hexenyl salicylate, linalyl acetate, methyl dihydrojasmonate, styralyl propionate, vetiveryl acetate, benzyl acetate, geranyl acetate;
lactones: such as γ-undecalactone, δ-decalactone, pentadecanolide, 12-oxahexadecanolide;
acetals: such as Viridine (phenylacetaldehyde dimethylacetal);
various components: often used in perfumery such as indole, p-mentha-8-thiol-3-one, methyleugenol, eugenol, anethol.

The novel odorants harmonize particularly well with all other floral notes (lily of the valley, rose, orris, jasmine, ylang-ylang, narcissus notes,), as well as with woody, chypre and animalic notes, tobacco like and patchouli compositions.

The percentage in which they are used in composition may vary within wide limits ranging from a few parts per thousand in mass market products (e.g. cleaning, deodorant) up to a few percent in alcoholic extracts for (fine) perfumery. In all cases, even in small amounts, they provide odorant compositions with intense floral notes and increase the volume (strength, diffusivity) and the substantivity of the odor. In particular, the manner in which they extend the diffusivity and the olfactory duration of the composition is remarkable.

There is really no restriction regarding the type of formulations and the destination of the actual finished product thus, eau de cologne, toilet water, scented water, perfume, cream, shampoo, deodorant, soap, detergent powder, household cleaner, fabric softener, come into consideration.

The invention will be further described, by way of illustration, in the following examples.

Convenient methods for preparing the compounds of the invention are outlined in the examples without limiting the invention thereto.

All compounds were unambiguously identified by their ¹H-NMR-, IR- and MS-spectra that were measured under the following conditions:
- IR: *Nicolet 510 FT-IR*; neat ; ν in cm⁻¹,
- ¹H NMR: *Bruker DPX-400*; at 250 and 400 MHz; in CDCl₃ if not otherwise stated; chemical shifts (*d*) in ppm downfield from TMS; coupling constants *J* in Hz,
- MS and GC/MS: *Finnigan MAT 212* (EI, 70eV); intensities (in brackets) in % rel. to the base peak.

They were always purified by fractional distillation, or *bulb-to-bulb* distillation if after flash chromatography *(Merck* silica gel 60; 230 - 400 mesh), and were olfactorily pure, colorless oils.

### Example 1

### 5-Cyclopentyl-3-methylpent-4-en-1-ol

A solution of ethyl 5-cyclopentyl-3-methylpent-4-enoate (16.8 g; 80 mmol; obtained according to Streinz, L.; Romanuk, M.; Sorm, F.; Sehnal, F. *DE 2 444 837*, priority 20.09.**1973**) in diethyl ether (30 ml) was added dropwise to a suspension of lithium aluminum hydride (3.0 g; 80 mmol) in the same solvent (110 ml), and the reaction mixture was stirred at reflux for 1 h. Water (3.5 ml), then 15% NaOH solution and again water (3.5 ml) were added, the precipitate filtered off and washed with MTBE (30 ml). The combined organic phases were washed with 1 *N* HCl (200 ml) and brine (3 x 100 ml), dried (MgSO₄), concentrated *in vacuo*, and distilled (79-82°C/9.33 Pa (0.07 torr)) to give 7.8 g (59 % yield) of 5-cyclopentyl-3-methylpent-4-en-1-ol.
IR: 3329, 2953, 2869, 1453, 1373, 1052, 999, 969. ¹H-NMR: 0.98 (*d*, *J* = 6.7, 3H), 1.16-1.40 (*m*, 2H), 1.46-1.82 (*m*, 9H), 2.12-2.28 (*m*, 1H), 2.26-2.47 (*m*, 1H), 3.64 (*t*, *J* = 6.6, 2H), 5.25 (*dd*, *J* = 15.3, 7.3, 1H), 5.41 (*dd*, *J =* 15.3, 7.0, 1H). MS: 168 (1.3, *M*^{*+*}), 150 (3), 135 (8), 121 (9), 108 (8), 107 (9), 95 (64), 93 (31), 82 (63), 81 (93), 79 (40), 69 (35), 67 (100), 55 (59), 41 (57).
Odor: floral, fruity, hesperidic/citrus, very strong and substantive.

### Example 2

### 5-Cyclopentyl-3-methylpent-4-enal

Diisobutylaluminum hydride (95 ml of 1.0 M solution in hexane) was added into a hexane (200 ml) solution of ethyl 5-cyclopentyl-3-methylpent-4-enoate (used in example 1; 20.0 g; 95 mmol), at -65°C. After 3 h stirring at the same temperature, ethanol (3 ml) was added, and the reaction mixture was poured into an ice-cold NH₄Cl solution (200 ml) and diluted with 2 *N* HCl (100 ml). The organic layer was separated, washed with brine (3 x 200 ml), dried (MgSO₄), concentrated *in vacuo*, and purified by flash chromatography (hexane/MTBE 15 :1) to give 10.9 g (69 % yield) of 5-cyclopentyl-3-methylpent-4-enal.
IR: 2954, 2870, 2716, 1727, 1453, 1375, 970. ¹H-NMR: 1.06 (*d*, *J* = 7.0, 3H), 1.12-1.36 (*m*, 2H), 1.44-1.83 (*m*, 6H), 2.25-2.49 (*m*, 3H), 2.61-2.81 (*m*, *J* = 6.7, 1H), 5.32 (*dd*, *J* = 15.4, 6.1, 1H), 5.44 (*dd*, *J* = 15.4, 6.4, 1H), 9.71 (*t*, *J* = 2.3, 1H). MS: 166 (1.5, *M*^{*+*}), 151 (3), 148 (3), 122 (61), 107 (15), 98 (43), 97 (57), 95 (44), 93 (64), 81 (63), 80 (36), 79 (47), 69 (47), 67 (100), 55 (71), 41 (88), 39 (41).
Odor: aldehydic, citrus, geranium.

### Example 3

### 5-Cyclopentyl-3-methylpent-4-en-1-yl acetate

Acetyl chloride (1.7 g; 22 mmol) was added to a cooled solution of 5-cyclopentyl-3-methylpent-4-en-1-ol (obtained in example 1; 2.6 g; 15 mmol), pyridine (2.4 g; 30 mmol), and DMAP (0.13 g; 0.1 mmol) in cyclohexane (65 ml). After 3 h stirring at r.t., the reaction mixture was poured into 1*N* HCl (130 ml) and MTBE (65 ml). The organic layer was separated, washed successively with 1*N* HCl (130ml), sodium bicarbonate solution (100 ml) and brine (2 x 100 ml) and treated as in example 1 (distillation at 120°C/106.66 Pa (0.8 torr)) to give 3.05 g (94 % yield) of 5-cyclopentyl-3-methylpent-4-en-1-yl acetate.
IR: 2954, 2869, 1743, 1454, 1366, 1238, 1048, 970. ¹H-NMR: 0.99 (*d*, *J =* 6.7, 3H), 1.13-1.34 (*m*, 2H), 1.44-1.83 (*m*, 8H), 2.04 (*s*, 3H), 2.08-2.30 (*m*, *J* = 7.0, 1H), 2.26-2.47 (*m*, 1H), 4.05 (*t*, *J* = 6.8, 2H), 5.20 (*dd*, *J =* 15.4, 7.3, 1H), 5.37 (*dd*, *J* = 15.4, 7.0, 1H). MS: 195 (0.1, *M*^{*+*} - CH₃), 150 (10), 135 (13), 121 (18), 108 (13), 107 (11), 95 (18), 93 (29), 82 (22), 81 (100), 80 (23), 79 (25), 67 (43), 55 (28), 41 (27).
Odor: fruity, pear, pineapple, floral.

### Example 4

### 5-Cyclopentyl-3-methylpentan-1-ol

Ethyl 5-cyclopentyl-3-methylpent-4-enoate of example 1 (22.4 g; 0.11 mol) was hydrogenated over 5% Pd/C in ethanol (220 ml), at r.t. and under atmospheric pressure. The catalyst was filtered off, the solvent evaporated *in vacuo* and the residue distilled (59°C/10.67 Pa (0.08 torr)) to give 19.5 g (92% yield) of 5-cyclopentyl-3-methylpentanoate that was reduced with lithium aluminum hydride as in example 1 to give 5-cyclopentyl-3-methylpentan-1-ol in 76.5% yield.
IR: 3331, 2949, 2867, 1454, 1377, 1059, 1010. ¹H-NMR: 0.89 (*d*, *J* = 6.4, 3H), 0.97-1.84 (*m*, 17H), 3.68 (*m*, 2H). MS: 152 (0.4, *M*^{*+*} - H₂O), 137 (11), 124 (21), 123 (16), 110 (12), 109 (14), 95 (85), 82 (100), 71 (28), 69 (72), 67 (77), 55 (77), 41 (51).
Odor: very strong, rosy, geranium, woody.

### Example 5

### 5-Cyclopentyl-3-methylpentanenitrile

### a) 5-Cyclopentyl-3-methylpentanal oxime

An aqueous (7 ml) solution of hydroxylamine hydrochloride (3.9 g; 56 mmol) was added to an ethanolic (20 ml) solution of 5-cyclopentyl-3-methylpentanal (8.0 g; 47 mmol), obtained from ethyl 5-cyclopentyl-3-methylpentanoate of example 4 by diisobutylaluminum hydride reduction according to example 2 (73% yield). The reaction mixture was heated to 50°C and treated with a solution of sodium hydroxide (2.7 g; 67 mmol) in water (5 ml). After 2 h stirring at r.t., ice (25 g) was added, and the mixture was saturated with carbon dioxide (solid). The organic layer was separated, dried (MgSO₄), concentrated *in vacuo*, and purified by flash chromatography (MTBE/hexane 1:4) to give 5.8 g (67% yield) of 5-cyclopentyl-3-methylpentanal oxime.

### b) 5-Cyclopentyl-3-methylpentanenitrile

5-cyclopentyl-3-methylpentanal oxime (3.7 g; 20 mmol) and acetic anhydride (4.5 g; 40 mmol) were heated at 110°C during 1.5 h, poured into ice-water (100 ml), and extracted with MTBE (150 ml). The organic phase was washed with brine (4 x 150 ml), dried (MgSO₄), concentrated *in* vacuo, and purified by flash chromatography (MTBE/hexane 1:15) to give 1.9 g (57% yield) of 5-cyclopentyl-3-methylpentanenitrile.
IR: 2950, 2866, 2246, 1457, 1425, 1384. ¹H-NMR: 1.06 (*d*, *J* = 6.7, 3H), 1.25-1.91 (*m*, 14H), 2.22 (*dd*, *J* = 16.7, 6.3, 1H), 2.33 (*dd*, *J* = 16.7, 6.0, 1H). MS: 165 (0.5, *M*^{*+*}), 164 (5), 150 (12), 136 (23), 124 (100), 122 (26), 109 (14), 97 (27), 94 (19), 83 (14), 82 (15), 69 (48), 68 (45), 55 (66), 41 (88).
Odor: citrus, geranitrile, peach, rosy.

### Example 6

### 4-Cyclopentylpentan-1-ol

### a) 4-Cyclopentylpentanoic acid

4-Cyclopenta-2,4-dienylidenepentanoic acid (25 g; 0.15 mol; obtained according to Coe, J. W.; Vetelino, M. G.; Kemp, D. S., *Tetrahedron Lett*., **1994**, *35*, 6627.) in ethyl acetate (270 ml) was hydrogenated as in example 4 to give 23.6 g (92% yield) of crude 4-cyclopentylpentanoic acid which was used in the next step without further purification.

### b) 4-Cyclopentylpentan-1-ol

A solution of 4-cyclopentylpentanoic acid (16 g; 94 mmol) in diethyl ether (30 ml) and THF (30 ml) was added within 20 min. to lithium aluminum hydride (3.6 g; 94 mmol) suspended in the same solvent (100 ml). After 2 h at reflux, the reaction mixture was cooled with an ice-bath and quenched successively with water (4 ml), 15% sodium hydroxide (12 ml) and again water (4 ml). The white solid was filtered off, and the mixture diluted with MTBE (300 ml), washed with 1 *N* HCl (300 ml), sodium bicarbonate solution (300 ml) and brine (2 x 300 ml), dried (MgSO₄), concentrated *in vacuo*, and distilled (59°C/10 Pa (0.075 torr)) to give 8.7 g (59 % yield) of 4-cyclopentylpentan-1-ol.
IR: 3329, 2950, 2867, 1451, 1377, 1056, 894. ¹H-NMR: 0.88 (*d*, *J =* 6.4, 3H), 1.0-1.81 (*m*, 15H), 3.62 (*m*, 2H);. MS: 138 (3, *M*^{*+*} - H₂O), 123 (3), 110 (42), 109 (14), 97 (58), 96 (40), 95 (35), 87 (24), 81 (27), 68 (38), 67 (51), 55 (60), 41 (43).
Odor: floral, woody, citrus, metallic.

### Example 7

### 4-Cyclopentylpent-1-yl propanoate

4-Cyclopentylpentan-1-ol (4.0 g; 23 mmol) was esterified with propionyl chloride (3.1 g; 34 mmol) according to example 3 to give 4.4 g (84.5% yield) of 4-cyclopentylpent-1-yl propanoate.
IR: 2951, 2868, 1743, 1456, 1366, 1239, 1048. ¹H-NMR: 0.87 (*d*, *J* = 6.4, 3H), 1.00-1.87 (*m*, 14H), 1.14 (*t*, *J* = 7.5, 3H), 2.32 *(q, J* = 7.6, 2H), 4.05 *(t, J* = 6.6, 2H). MS: 226 (0.01, *M*^{*+*}), 197 (4), 152 (10), 137 (12), 124 (17), 123 (18), 110 (22), 96 (32), 95 (88), 83 (50), 82 (100), 81 (42), 75 (40), 69 (44), 67 (59), 57 (67), 55 (53), 41 (32).
Odor: orange, fruity, ozonic, floral.

### Example 8

### Ethyl 4-cyclopentylpentanoate

1,1'-Carbonyldiimidazole (101 g; 0.62 mmol) was added portionwise to 4-cyclopentylpentanoic acid (100 g; 0.58 mol) in THF (500 ml). After the gas evolution ceased, the reaction mixture was reacted with sodium ethylate solution prepared from sodium (0.3 g; 13 mmol) and ethanol (500 ml), stirred at r.t. for 2.5 h, and the solvent evaporated *in vacuo*. The residue was dissolved in ether (300 ml), washed successively with water (300 ml), 1 *N* NaOH (300 ml), and 1 *N* HCl (300 ml), dried (MgSO₄), concentrated *in vacuo,* and distilled over a 10 cm Vigreux column (66°C/10.67 Pa (0.08 torr)) to give 59.4 g (52% yield) of ethyl 4-cyclopentylpentanoate.
IR: 2953, 2869, 1738, 1451, 1376, 1253, 1181, 1104, 1037, 939. ¹H-NMR: 0.87 (*d*, *J* = 6.4, 3H), 1.26 (*t*, *J* = 7.2, 3H), 1.01-1.91 (*m*, 12H), 2.23 (*ddd*, *J* = 15.3, 8.8, 6.4, 1H), 2.37 (*ddd*, *J* = 15.3, 9.8, 5.5, 1H), 4.12 *(q, J* = 7.1, 2H). MS: 183 (0.1, *M*^{*+*} - CH₃), 153 (3), 141 (16), 135 (32), 129 (23), 111 (65), 110 (31), 101 (98), 88 (85), 69 (49), 67 (36), 55 (100), 41 (67).
Odor: green, fruity (pineapple), floral (rosy).

### Example 9

### 4-Cyclopentylpentanal

Ethyl 4-cyclopentylpentanoate was reduced to 4-cyclopentylpentanal with diisobutylaluminum hydride as in example 2 (68% yield).
IR: 2952, 2868, 2714, 1727, 1450, 1411, 1379, 1012. ¹H-NMR: 0.88 (*d*, *J* = 6.4, 3H), 1.01-1.91 (*m*, 12H), 2.29-2.57 (*m*, 2H), 9.77 (*t*, *J*= 2.0, 1H). MS: 154 (4, *M*^{*+*}), 139 (10), 136 (18), 121 (19), 110 (77), 97 (44), 95 (53), 85 (36), 81 (35), 69 (62), 68 (76), 67 (100), 55 (76), 41 (60).
Odor: aldehydic, melon, mandarine, green ivy, floral.

### Example 10

### 4-Cyclopentylpentanenitrile

4-Cyclopentylpentanal was transformed into 4-cyclopentylpentanenitrile as in example 5 (56% yield).
IR: 2953, 2869, 2246, 1450, 1428, 1381. ¹H-NMR: 0.91 (*d*, *J* = 6.1, 3H), 1.04-1.26 (*m*, 2H), 1.36-1.93 (*m*, 10H), 2.21-2.49 (*m*, 2H). MS: 151 (0.1, *M*^{*+*}), 150 (0.5), 136 (4), 123 (3), 110 (45), 109 (43), 97 (11), 83 (18), 69 (63), 68 (19), 67 (21), 55 (83), 41 (100).
Odor: hesperidic, floral, green, cumin.

### Example 11

### 5-Cyclopentylhexanenitrile

### a) 5-(Cyclopenta-2,4-dienylidene)hexanenitrile

Pyrrolidine (18.5 g; 0.26 mol) was added to a solution of cyclopentadiene (10.8 g; 0.16 mol; freshly prepared by cracking of dicyclopentadiene) and 5-oxohexanenitrile (15.4 g; 0.13 mol) in methanol (175 ml) at 0°C. After 1 h stirring at this temperature, the reaction mixture was poured into ice-cold 2 *N* HCl (500 ml), saturated with sodium chloride and extracted with MTBE (400 ml). The organic phase was washed with brine (3 x 300 ml), dried (MgSO₄), concentrated *in vacuo*, and used in the next step without further purification.

### b) 5-Cyclopentylhexanenitrile

Hydrogenation as in example 4 gave 5-cyclopentylhexanenitrile.
IR: 2952, 2868, 2245, 1458, 1427, 1378. ¹H-NMR: 0.88 (*d*, *J* = 6.1, 3H), 1.01-1.86 (*m*, 14H), 2.28-2.37 (*m*, 2H). MS: 165 (2, *M*^{*+*}), 164 (12), 150 (25), 136 (17), 124 (67), 122 (39), 98 (61), 97 (100), 96 (47), 82 (34), 69 (80), 68 (38), 67 (25), 55 (71), 41 (49).
Odor: floral, orris, spicy, powdery, cumin.

### Example 12

### 5-Cyclopentylhexan-2-one

4-Cyclopentylpentanoic acid (3.4 g; 20 mmol; prepared in example 6) in diethyl ether (90 ml) was added at 0°C to 1.6 *M* diethyl ether solution of methyllithium (25 ml; 40 mmol), diluted with the same solvent (75 ml). After 3.5 h stirring at 5°C, water (100 ml) was added, and the separated organic layer was washed with brine (3 x 400 ml), dried (MgSO₄), concentrated *in vacuo*, and bulb-to-bulb distilled (125°C/26.66 Pa (0.2 torr)) to give 1.8 g (53.5 yield) of 5-cyclopentylhexan-2-one.
IR: 2952, 2868, 1718, 1450, 1412, 1357, 1162. ¹H-NMR: 0.86 (*d*, *J* = 6.1, 3H), 1.02-1.85 (*m*, 12H), 2.15 (*s,* 3H), 2.28-2.57 (*m*, 2H). MS: 168 (5, *M*^{*+*}), 150 (12), 135 (21), 121 (96), 111 (79), 110 (70), 108 (46), 81 (27), 71 (46), 69 (58), 67 (58), 58 (55), 55 (57), 43 (100), 41 (35).
Odor: fruity, lavender, orris, lactonic, pineapple.

### Example 13

### 5-Cyclopentylidenehexanenitrile and 5-(cyclopent-1-enyl)-hexanenitrile

### a) 5-Cyclopentyl-5-hydroxyhexanenitrile

5-Oxohexanenitrile (11.1 g; 0.10 mmol) was added at 20 °C to a suspension of anhydrous cerium(III) chloride (30 g; 0.12 mol) in THF (250 ml). After 1 h stirring at r.t., cyclopentylmagnesium chloride (60 ml of 2.0 *M* solution in diethyl ether; 0.12 mol) was added dropwise at 5°C and stirring continued for more 0.5 h at the same temperature. 2 *N* HCl (60 ml) was added, and the reaction mixture was extracted with MTBE (2 x 200 ml). The combined organic phases was washed with 2 *N* HCl (100 ml), and brine (2 x 100 ml), dried (MgSO₄), and concentrated *in vacuo*, to give 13 g of crude (83% GC pure; 60% yield) 5-cyclopentyl-5-hydroxyhexanenitrile, used without further purification in the next step.

### b) 5-Cyclopentylidenehexanenitrile and 5-(cyclopent-1-enyl)hexanenitrile

Crude 5-cyclopentyl-5-hydroxyhexanenitrile (18 g; 0.1 mol) was added into a solution of sulphuric acid (15 ml) in acetic acid (150 ml). The reaction mixture was stirred at 5°C for 1 h, then poured into ice (100 g), diluted with MTBE (100 ml), washed with saturated sodium bicarbonate solution (5 x 300 ml), and brine (2 x 300 ml), dried (MgSO₄), concentrated *in vacuo*, and bulb-to-bulb distilled to give 2.1 g (13 % yield) of 5-cyclopentylidene-hexanenitrile and 5-(cyclopent-1-enyl)-hexanenitrile mixture (GC: 43 + 57%).
IR: 2953, 2867, 2245, 1457, 1433, 1377. ¹H-NMR: 1.03 (*d*, *J* = 6.7, 1.5H), 1.41-1.66 (*m*, 4H), 1.60 (*s*, 1.5H), 1.75 (*m*, *J* = 7.3, 1H), 1.84 (*m*, *J* = 7.4, 1H), 2.11-2.38 (*m*, 7.5H), 5.36 (*m*, 0.5H). MS (major product): 163 (26, *M*^{*+*}), 148 (62), 135 (20), 134 (23), 120 (32), 107 (66), 95 (77), 91 (24), 79 (32), 77 (25), 67 (100), 55 (29), 41 (42); MS (minor product): 163 (13, *M*^{*+*}), 163 (6), 148 (28), 135 (18), 134 (12), 120 (38), 107 (31), 95 (100), 91 (18), 79 (24), 77 (20), 67 (93), 55 (20), 41 (33).
Odor: cumin, orris, spicy, floral, stronger than 5-cyclopentylhexanenitrile.

### Example 14

### 5-Cyclopentylhexanal

Diisobutylaluminum hydride (60 ml of 1.0 *M* solution in hexane) was added at -65°C to a hexane (50 ml) solution of 5-cyclopentylhexanenitrile (5.0 g; 30 mmol; from example 11). After stirring at -70°C for 0.5 h, and at r.t. for 3 h, methanol (1.8 ml) was added, and the stirring continued for 20 min.. 10% H₂SO₄ (48 ml) was added, and the reaction mixture was diluted with MTBE (150 ml). The organic layer was separated, washed with saturated sodium bicarbonate solution (300 ml), and brine (3 x 300 ml), dried (MgSO₄), concentrated *in vacuo*, and bulb-to-bulb distilled (100°C/26.66 Pa (0.2torr)) to give 2.6 g (51.5 % yield) of 5-cyclopentylhexanal.
IR: 2950, 2867, 2715, 1727, 1452, 1410, 1377. ¹H-NMR: 0.90 (*d*, *J* = 6.4, 3H), 1.01-1.91 (*m*, 14H), 2.35-2.46 (*m*, 2H), 9.77 (*t*, *J* = 1.8, 1H). MS: 168 (19, *M*^{*+*}), 150 (1), 135 (27), 121 (19), 109 (29), 97 (57), 96 (100), 95 (34), 81 (65), 69 (50), 68 (43), 67 (56), 55 (98), 41 (48).
Odor: aldehydic, green, fresh, hesperidic, linear.

### Example 15

### 5-Cyclopentylhexan-1-ol

5-Cyclopentylhexanal (4.2 g; 25 mmol) in ethanol (40 ml) was added to sodium borohydride (1.2 g; 32 mmol) suspended in the same solvent (50 ml), at 10°C, and the reaction mixture was stirred at r.t. for 2 h. 1 *N* HCl (50 ml) was added dropwise at 0°C. The mixture was diluted with MTBE (150 ml), the organic layer separated, washed with brine (3 x 250 ml), dried (MgSO₄), concentrated *in vacuo*, and bulb-to-bulb distilled (125°C/26.66 Pa (0.2 torr)) to give 3.4 g (80 % yield) of 5-cyclopentylhexan-1-ol.
IR: 3326, 2949, 2865, 1452, 1376, 1059. ¹H-NMR: 0.86 (*d*, *J* = 6.4, 3H), 1.01-1.82 (*m*, 17H), 3.63 (*t*, *J =* 6.5, 2H). MS: 152 (0.7, *M*^{*+*} - H₂O), 137 (3), 123 (4), 110 (14), 109 (18), 101 (14), 97 (72), 96 (49), 95 (28), 83 (80), 82 (53), 69 (33), 68 (33), 67 (47), 55 (100), 41 (35).
Odor: floral, sweet, fruity, raspberry, powdery, rosy.

### Example 16

### 5-(2-Methylcyclopent-1-enyl)pentan-1-ol and 5-(5-methyl-cyclopent-1-enyl)pentan-1-ol

1,4-Dibromopentane (76 g; 0,33 mol) dissolved in THF (450 ml) was added at reflux within 70 min. to magnesium turnings (15.8g; 0,66 mol) in THF (50 ml). The mixture was refluxed for 90 min., cooled to room temperature and diluted with THF (250 ml). This solution was added within 160 min. to caprolactone (37.6 g; 0,33 mol) dissolved in THF (400 ml), the temperature being kept at 5°C. After further stirring for 90 min. without cooling, the mixture was poured into ice-water (1.2 l), acidified to pH 2 (HCl) and extracted with MTBE (2 x 600 ml). The organic phases were washed with water (4 x 800 ml), dried (Na₂SO₄), concentrated *in vacuo* and distilled (2.67 Pa (0.02 torr)) over KHSO₄ (1.3 g) to give 23 g of an oil which was further purified by flash-chromatography (MTBE/hexane 1:5) to give 4.8 g (9% yield) of a mixture of the two isomers.
IR: 3334, 2930, 2856, 1456, 1379, 1072, 1052. ¹H-NMR: 0.99 (*d*, J = 8, ca. 0,85H), 1.23-1.50 (*m*, ca. 5H), 1.52-1.69 (*m*, ca. 5H), 1.72-1.79 (*m*, 1H), 1.91-2.32 (*m*, 5H, 3.58-3.66 (*m*, 2H), 5.29 (*bs,* ca. 0.3H). MS: 168(18, *M*^{*+*}), 81 (1), 95 (2), 67 (3), 55(4), 41 (5), 107 (6), 135 (7), 121 (8).
Odor: floral, rosy, fruity (melon), marine.

### Example 17

### 4-(Cyclopent-1-enyl)butan-1-ol and 4-cyclopentylidene-butan-1-ol

1,4-Dibromobutane (64.8 g; 0.3 mol) dissolved in THF (300 ml) was added within 45 min. to magnesium (14.6 g; 0,6 mol) in the same solvent (35 ml). After 3h stirring at reflux, the solution was cooled to r.t., diluted with THF (30 ml) and added within 30 min. to δ-valerolactone (30 g; 0,3 mol) dissolved in THF (450 ml), the temperature being kept at 10°. After stirring at r.t. for 2h, the reaction mixture was poured into ice-water (500 ml), acidified to pH 2 (10% HCl) and extracted with MTBE (3 x 200 ml). The organic phases were washed with water (3 x 500 ml) dried (Na₂SO₄), concentrated *in vacuo* and distilled (13.33 Pa (0.1 Torr)) over KHSO₄ (0.5 g) to afford 10.5 g of an oil which after flash-chromatography (MTBE/hexane 1:5) gave 3.4 g (8% yield) of the two isomers.
IR: 3334, 2934, 2844, 1652, 1436, 1056, 1032. ¹H-NMR: 1.46-1.69 (*m*, ca. 4.5H), 1.8-1.89 (*m*, ca. 1.5H), 2.0-2.12 (m, 2H), 2.14-2.32 (*m*, 4H), 2.78 (*bm*, 1H), 3.61 (*t*, J = 7, 2H), 5.24 (*m*, ca. 0.25H), 5.33 (*t*, J = 1, ca. 0,75H); MS: 140 (19, *M*^{*+*}), 79(1), 67 (2), 93 (3), 41 (4), 53 (5), 31 (6), 107 (7), 122 (8).
Odor: rosy, aldehydic, green.

### Example 18

### 6-Cyclopentyl-3-methylhexan-3-ol

### a) 1-Ethenylcyclopentanol

A solution of vinylbromide (146.6 g; 1.37 mol) in 250 ml of THF was added during 4 h to a mixture of magnesium fine turnings (33.3 g; 1.37 mol) and a crystal of iodine in 150 ml of THF at t <50°C. The resulting dark grey mixture was stirred for 1 h, treated with a solution of cyclopentanone (104.8 g; 1.25 mol) in 100 ml of THF at 35- 45°C, and stirred overnight at r.t.. Saturated NH₄Cl solution(1 l) was added at ∼0°C and the reaction mixture was acidified to pH ∼6-7 with 2*N* HCl. The organic layer was separated, washed with brine (3 x 100 ml), dried over MgSO₄ and concentrated *in vacuo*. Distillation using a Widmer column (32°C/8 Pa (0.06 torr) yielded 67.6 g (48% yield) of 1-ethenylcyclopentanol.

### b) 5-Cyclopentylidenepentan-2-one

A 600 ml autoclave containing 1-ethenyl-cyclopentanol (172.0 g; 1.54 mol), isopropenyl methyl ether (218.8 g; 3.04 mol), triethylamine (1.65 ml) and 85% H₃PO₄ (0.72 ml) was pressurised with N₂ at 2 bar and heated up to 125°C. An increase of the pressure to 7 bar was observed. After 14 h stirring, the autoclave was cooled down to r.t. and depressurised. The mixture was taken in MTBE (1.5 l), washed with H₂O (4 x 25 ml) until neutral pH, dried (MgSO₄) and concentrated. Distillation (61°C/13.33 Pa (0.1torr)) yielded 161.3 g (69% yield) of 5-cyclopentylidenepentan-2-one.

### c) 6-Cyclopentylidene-3-methylhex-1-yn-3-ol

Acetylene was bubbled for 50 min. through a solution of tBuOK (33.8 g; 0.30 mol) in THF (240 ml), cooled down to 0°C. The resulting beige suspension was treated with 5-cyclopentylidenepentan-2-one (41.7 g; 0.27 mol) added dropwise for 15 min. at 0°C. The resulting mixture was warmed gently to r.t. and quenched with sat. NH₄Cl (180 ml). The aqueous phase was separated and extracted with MTBE (2 x 120 ml). The combined organic layers were washed with H₂O (240 ml), brine (100 ml), dried over MgSO₄ and concentrated *in vacuo*. Distillation (69-71°C/12 Pa (0.09 torr)) afforded 38.95 g (81% yield) of 6-cyclopentylidene-3-methylhex-1-yn-3-ol.

### d) 6-Cyclopentyl-3-methylhexan-3-ol

Hydrogenation of 6-cyclopentylidene-3-methylhex-1-yn-3-ol (5.0 g, 28 mmol) under standard conditions: H₂ 1 atm, r.t., over 5% Pd/C (0.57 g) in EtOH (30 ml) afforded after distillation (56°C/8 Pa (0.06 torr)) 4.57 g (89% yield) of 6-cyclopentyl-3-methylhexan-3-ol.
IR (neat): 3378, 2942, 2866. ¹H NMR: 0.89 (*t*, *J* = 7.5 Hz, 3H), 1.00-1.13 (*m*, 2H), 1.14 (*s*, 3H), 1.48 (*q*, *J* = 7.5 Hz, 2H), 1.24-1.65 (*m*, 11H), 1.69-1.84 (*m*, 3H). MS: 169 (2, *M*^{*+*} - CH₃), 155 (6), 137 (8), 95 (32), 81 (23), 73 (100), 67 (14), 55 (32), 43 (18), 41 (18).
Odor: floral (rosewood), fruity (apricot), hesperidic, neroli.

### Example 19

### 5-(Cyclopent-1-enyl)-2-methylpentan-2-ol

### a) 5-(Cyclopent-1-enyl)-2-pentanone

A mixture of 5-cyclopentylidene-2-pentanone (9.92 g; 65 mmol) and p-TsOH (100 mg; 0.53 mmol) in toluene (150 ml) was heated at 90°C for 8 h, then cooled down to r.t., diluted with MTBE (100 ml), washed with sat. NaHCO₃ (50 ml), H₂O (50 ml), brine (50 ml), dried over MgSO₄ and concentrated *in vacuo*. Distillation under reduced pressure (48°C/8.67 Pa (0.065 torr) yielded 7.13 g of 5-(cyclopent-1-enyl)-2-pentanone (purity ∼80%), further purified by flash chromatography to give 6.1 g (61% yield) of 89% pure product containing 11% of 5-cyclopentylidene-2-pentanone.

### b) 5-(Cyclopent-1-enyl)-2-methylpentan-2-ol

5-(Cyclopent-1-enyl)-2-pentanone (5.95 g; 39 mmol) in ethyl ether (6 ml) was added dropwise to a 3*M* solution of methylmagnesium bromide in the same solvent (17 ml; 51 mmol) during 15 min.. After 2 h at reflux, the mixture was cooled down to r.t., poured into ice (20 g), acidified with 5*N* HCl (20 ml) and extracted with MTBE (60 ml). The aqueous phase was separated and extracted again with MTBE (2 x 100 ml). The combined organic phases were washed with sat. NaHCO₃ (80 ml), H₂O (80 ml), brine (80 ml), dried over MgSO₄ and concentrated *in vacuo*. Bulb to bulb distillation (80°C/8 Pa (0.06 torr)) yielded quantitatively 5.84 g of 5-(cyclopent-1-enyl)-2-methylpentan-2-ol containing 11% of 5-cyclopentylidene-2-methylpentan-2-ol.
IR: 3364, 2967, 2939, 2867, 2844, 1468, 1377, 1296, 1195, 1149, 1047, 910, 772. ¹H NMR: 1.21 (*s*, 6H), 1.41-1.56 (*m*, 4H), 1.80-1.90 (*m*, 2H), 2.02-2.11 (*m*, 2H), 2.18-2.26 (*m*, 2H), 2.26-2.33 (*m*, 2H), 5.31-5.35 (*m*, 1H). MS: 168 (0.5, *M*^{*+*}), 150 (28), 135 (50), 95 (25), 94 (100), 93 (16), 81 (12), 80 (14), 79 (91), 69 (22), 67 (22), 59 (34), 43 (10), 41 (12).
Odor: floral (rosy, geranium), fruity (plum), agrestic.

### Example 20

### 5-Cyclopentylpentan-2-ol

### a) 5-Cyclopentylpentan-2-one

Hydrogenation of 5-cyclopentylidenepentan-2-one synthesized in example 18b (30.4 g; 0.2 mol) under standard conditions (cf. example 19d) gave, after distillation (61-67°C/13.33 Pa (0.1torr)), 25.8 g (84% yield) of 5-cyclopentylpentan-2-one.

### b) 5-Cyclopentylpentan-2-ol

NaBH₄ (3.15 g; 83 mmol) was added portionwise to a solution of 5-cyclopentylpentan-2-one (16.0 g; 104 mmol) in MeOH (125 ml) at 0°C. The mixture was stirred at 0°C for 1 h then at r.t. for 2 h. Water (100 ml) was added cautiously and the mixture extracted with MTBE (3 x 250 ml). The organic phases were combined, washed with brine (3 x 50 ml), dried over MgSO₄, concentrated *in vacuo* and distilled (52-56°C/6 Pa (0.045 torr)) to give 14.5 g (90% yield) of 5-cyclopentylpentan-2-ol.
IR: 3347, 2947, 2861, 1453, 1374, 1308, 1116, 1077, 942. ¹H NMR: 1.00-1.12 (*m*, 2H), 1.18 (*d*, *J* = 6.0, 3H), 1.23-1.38 (*m*, 3H), 1.38-1.64 (*m*, 7H), 1.67 (*bs*, 1H), 1.70-1.80 (*m*, 3H), 3.73-3.85 (*m*, 1H). MS: 141 (6, *M*^{*+*} - CH₃), 123 (18), 111 (16), 110 (22), 96 (52), 95 (48), 83 (28), 82 (70), 81 (60), 69 (42), 68 (36), 67 (84), 58 (12), 55 (34), 45 (100), 43 (16), 41 (35), 39 (12).
Odor: floral (tuberose), lily of the valley, coconut, celery.

### Example 21

### 5-Cyclopentylidene-2-methylpentan-2-ol

5-Cyclopentylidene-2-pentanone (13.4 g; 88 mmol) in ether (13 ml) was added dropwise during 30 min. to a 3*M* solution of methylmagnesium bromide in ether (38 ml; 114 mmol). After addition of more ether (25 ml), the mixture was heated under reflux for 2h, then cooled down to r.t., poured into ice (40 g), acidified with 5*N* HCl (50 ml) and extracted with MTBE (130 ml). The aqueous phase was separated and extracted with MTBE (2 x 130 ml). The combined organic phases were washed with sat. NaHCO₃ (2 x 130 ml), water (130 ml), dried over MgSO₄ and concentrated *in vacuo*. Distillation (52-55°C/8 Pa (0.06 torr)) yielded 11.24 g (76% yield) of 5-cyclopentylidene-2-methylpentan-2-ol.
IR: 3366, 2959, 2867, 1451, 1377, 1218, 1147, 910. ¹H NMR: 1.21 (*s*, 6H), 1.48-1.70 (*m*, 6H), 2.00-2.09 (*m*, 2H), 2.15-2.24 (*m*, 4H), 5.20-5.28 (*m*, 1H). MS: 168 (0.5, *M*^{*+*}), 150 (65), 135 (100), 121 (12), 107 (26), 95 (46), 94 (68), 93 (32), 82 (30), 81 (13), 80 (10), 79 (63), 67 (40), 59 (42), 55 (10), 43 (12), 41 (16).
Odor: floral ionone, linalool, raspberry, agrestic, tea.

### Example 22

### 5-Cyclopentylidenepent-2-yl propanoate

### a) 5-Cyclopentylidenepentan-2-ol

5-cyclopentylidenepentan-2-one (22.7 g; 149 mmol) was reduced as in example 20b to give 24.5 g of crude 5-cyclopentylidenepentan-2-ol that was used without further purification in the following step.

### b) 5-Cyclopentylidenepent-2-yl propanoate

DMAP (0.03 g; 0.24 mmol, 0.8) was added to a mixture of 5-cyclopentylidenepentan-2-ol (5.0 g; 32 mmol), propionic anhydride (6.7 ml, 52 mmol) and Et₃N (7.2 ml, 52 mmol). After 1.5 h stirring at r.t., the reaction mixture was diluted with MTBE (140 ml), washed with 2*N* HCl (60 ml), water (60 ml), sat. NaHCO₃ (60 ml), again with water (60 ml), and brine (60 ml), dried over MgSO₄ and concentrated *in vacuo*. Distillation under reduced pressure (87-92°C/5.33 Pa (0.04 torr)) afforded 5.38 g (80% yield) of 5-cyclopentylidenepent-2-yl propanoate.
IR: 2943, 2868, 1736, 1462, 1370, 1192, 1129, 1082. ¹H NMR: 1.14 (*t*, *J* = 7.6, 3H), 1.21 (*d*, *J* = 6.0, 3H), 1.46-1.70 (*m*, 6H), 1.91-2.07 (*m*, 2H), 2.10-2.18 (*m*, 2H), 2.18-2.25 (*m*, 2H), 2.30 (*q*, *J* = 7.6, 2H), 4.85-4.95 (*m*, 1H), 5.17-5.25 (*m*, 1H). MS: 195 (0.5, *M*^{*+*} - CH₃), 136 (100), 121 (57), 107 (60), 95 (54), 94 (72), 93 (66), 91 (14), 81 (18), 80 (28), 79 (73), 77 (11), 68 (31), 67 (35), 57 (34), 41 (15).
Odor: floral, fruity, pear, ionone, apple.

### Example 23

### Shower gel perfume

| | |
|---|---|
| **5-Cyclopentylhexanenitrile** 10% DPG | 7.5 |
| Aldehyde C 12 lauric 10% DPG | 10 |
| Ambrettolide | 10 |
| Benzyl acetate | 30 |
| Bergamote abergapt oil | 115 |
| Berryflor | 3 |
| Cetone alpha | 40 |
| Citronellol E | 20 |
| Citronellyl acetate | 10 |
| Coumarin pure crist. | 20 |
| Dipropylene glycol | 117 |
| Ethyl linalool | 35 |
| Ethyl vanillin | 5 |
| Ethylene brassylate | 130 |
| Eucalyptol | 4 |
| Eugenol pur | 5 |
| Givescone | 3 |
| Hexyl cinnamic aldehyde | 200 |
| Ionone beta | 10 |
| Lemonile | 3 |
| Lilial | 30 |
| Myraldene | 3 |
| Orange Floride oil | 30 |
| Prunolide | 10 |
| Radjanol | 40 |
| Rhodinol 70 | 10 |
| Tricyclal | 3 |
| Tuberose base (reconstitution) | 7.5 |
| Verdantiol | 4 |
| Vertofix coeur | 50 |
| Ylang ylang oil | 35 |
| | 1000 |

5-Cyclopentylhexanenitrile brings a lot of diffusion to the fragrance, imparting a very rich orris, ionone effect to the composition; the floral rosy/lily of the valley part of the fragrance is also considerably enhanced; this compound brings both power and elegance to this shower gel perfume.

### Example 24

### Fine fragrance for men

| | |
|---|---|
| **5-Cyclopentylhexan-1-ol** 10% DPG | 30 |
| Amyris oil | 50 |
| Benzoin tears Siam 50% DEP | 50 |
| Bergamote Italy oil | 175 |
| Cepionate | 60 |
| Citronellyl acetate | 25 |
| Damascenone 10% DPG | 10 |
| Dihydrolinalool | 50 |
| Dimethylbenzylcarbinol isobutyrate | 6 |
| Dimetol | 40 |
| Fennaldehyde | 15 |
| Fixambrene | 4 |
| Florhydral | 5 |
| Florol | 50 |
| Gardenol | 5 |
| Laurine | 80 |
| Lemon Italy oil | 40 |
| Magnolione | 80 |
| Mandarine green Italy oil | 10 |
| Olibanum res. 50% DEP | 45 |
| Orange Florida oil | 60 |
| Thibetolide | 10 |
| Tricyclal 10% DPG | 25 |
| Tropional | 40 |
| Undecavertol | 15 |
| Velvione | 20 |
| | 1000 |

5-Cyclopentylhexan-1-ol brings on the whole higher diffusion to the blend. It enhances hesperidic notes, particularly mandarine effect. The aldehydic rosy effect it imparts adds to the transparency of the fragrance and to its modernity through marine undertones.

### Example 25

### Deodorant fragrance

| | |
|---|---|
| **5-Cyclopentylhexanal** 10% DPG | 30 |
| Bergamote base | 50 |
| Cedryl acetate | 25 |
| Cepionate | 50 |
| Coumarin | 5 |
| Cyclohexyl salicylate | 12 |
| Dipropylene glycol | 105 |
| Dynascone 10 | 15 |
| Elemi oil | 5 |
| Fixambrene | 5 |
| Folrosia | 6 |
| Hydro Rose C (rose oil reconstitution) | 300 |
| Iso E Super | 85 |
| Junniper berries oil | 20 |
| Kephalis | 30 |
| Lemon Italy oil | 50 |
| Mate absolute 10%DPG | 35 |
| Metambrate | 45 |
| Methyl Pamplemousse | 8 |
| Nutmeg oil | 8 |
| Okoumal | 10 |
| Patchouli SF oil | 8 |
| Sandalore | 35 |
| Spearmint USA 10%DPG | 20 |
| Thibetolide | 30 |
| | 1000 |

**5-Cyclopentylhexanal** brings higher harmony to the blend; it imparts anisic, tarragon type of nuances, along with its natural rosy/geranium effect; through evaporation the anisic undertones evolve towards a floral, marine, anisic cocktail which underlines the modern transparent and clean effect of the fragrance in the deodorant application.

For the exact definition of the trivial names mentioned above, see *Flavor and Fragrance Materials 1998*, Allured Publishing Corporation, Carol Stream, Illinois, U.S.A. or Arctander, Perfume and Flavor Chemicals - 1969, published by the author, Montclair, New Jersey, U.S.A.

## Claims

1. Compounds of general formula I 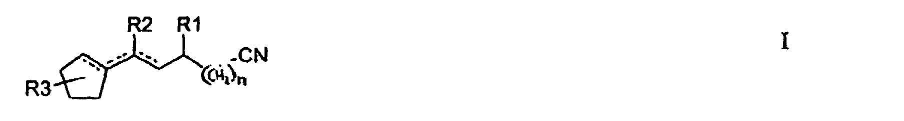 wherein
R¹, R² and R³ are independently H, C₁₋₃ alkyl but R¹ and R² are not at the same time H;
R² can also be methylene or ethylidene;
n = 0 or 1 and
--- stands for a single or a double bond, whereby maximum 2 double bonds are present

2. 5-Cyclopentyl-3-methylpentanenitrile according to claim 1.

3. 4-Cyclopentylpentanenitrile according to claim 1.

4. 5-Cyclopentylhexanenitrile according to claim 1.

5. 5-Cyclopentylidenehexanenitrile accordidng to claim 1.

6. 5-(Cyclopent-1-enyl)hexanenitrile according to claim 1.

7. Use of the compounds of general formula II 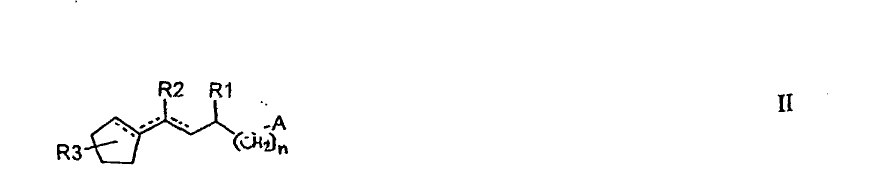 as fragrance
wherein
A is selected from the group of CR⁴R⁵OH, CR⁴R⁵OC(O)R⁶, CO₂R⁶, and CN;
R¹, R², R³, R⁴, R⁵ and R⁶ are independently H or C₁₋₃-alkyl;
R² can also be methylene or ethylidene;
R⁶ can also be a C₂₋₄ alkenyl or alkinyl
n = 0 or 1 and
--- stands for a single or a double bond, whereby maximum 2 double bonds are present,.

8. Use of 5-cyclopentyl-3-methylpent-4-enal, 4-cyclopentylpentanal, or 5-cyclopentylhexanal as fragrance.

9. Use according to claim 7 and 8 in fine and functional perfumery.

## Patentansprüche

1. Verbindungen der allgemeinen Formel I 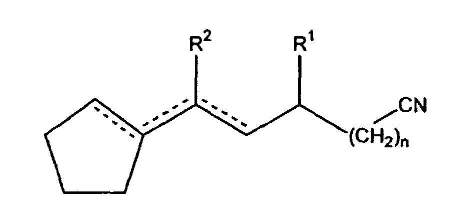 in der R¹, R² und R³ unabhängig voneinander H, C₁₋₃ Alkyl sind, aber R¹ und R² nicht gleichzeitig H sind;
R² ebenfalls Methylen oder Ethyliden sein kann;
n = 0 oder 1 ist und
--- für eine Einfach- oder eine Doppelbindung steht, wobei maximal 2 Doppelbindungen anwesend sind.

2. 5-Cyclopentyl-3-methylpentannitril nach Anspruch 1.

3. 4-Cyclopentylpentannitril nach Anspruch 1.

4. 5-Cyclopentylhexannitril nach Anspruch 1.

5. 5-Cyclopentylidenhexannitril nach Anspruch 1.

6. 5-(Cyclopent-1-enyl)hexannitril nach Anspruch 1.

7. Verwendung der Verbindungen der allgemeinen Formel II 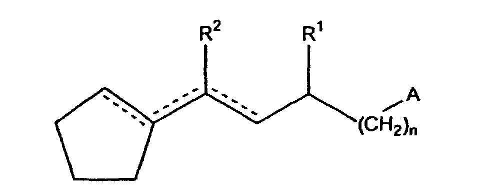 als Duftstoff, in der A ausgewählt ist aus der Gruppe von CR⁴R⁵OH, CR⁴R⁵OC(O)R⁶, CO₂R⁶ und CN;
R¹, R², R³, R⁴, R⁵ und R⁶ unabhängig voneinander H oder C₁₋₃-Alkyl sind,
R² ebenfalls Methylen oder Ethyliden sein kann;
R⁶ auch ein C₂₋₄ Alkenyl oder Alkinyl sein kann
n = 0 oder 1 ist und
--- für eine Einfach- oder eine Doppelbindung steht, wobei maximal 2 Doppelbindungen anwesend sind.

8. Verwendung von 5-Cyclopentyl-3-methylpent-4-enal, 4-Cyclopentylpentanal oder 5-Cyclopentylhexanal als Duftstoff.

9. Verwendung nach Anspruch 7 und 8 in der Feinparfümerie und der funktionellen Parfümerie.

## Revendications

1. Composés de formule générale I 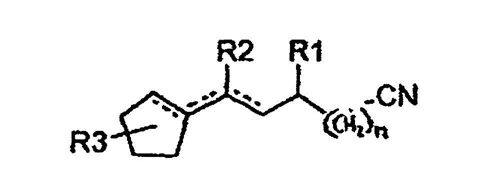 dans laquelle
R¹, R² et R³ sont indépendemment H, un alkyle en C₁₋₃, mais R¹ et R² ne sont pas simultanément H ;
R² peut également être un méthylène ou un éthylidène ;
m vaut 0 ou 1 et
--- représente une simple ou double liaison, 2 doubles liaisons étant présentes au maximum.

2. 5-Cyclopentyl-3-méthylpentanenitrile selon la revendication 1.

3. 4-Cyclopentylpentanenitrile selon la revendication 1.

4. 5-Cyclopentylhexanenitrile selon la revendication 1.

5. 5-Cyclopentylidènehexanenitrile selon la revendication 1.

6. 5-(Cyclopent-1-ényl)hexanenitrile selon la revendication 1.

7. Utilisation des composés de formule générale II 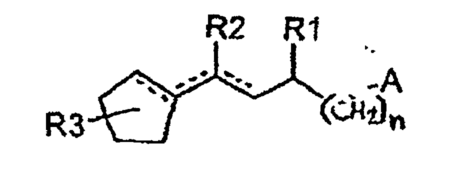 en tant que parfum,
dans laquelle
A est choisi parmi le groupe constitué de CR⁴R⁵OH, de CR⁴R⁵OC(O)R⁶, de CO₂R⁶ et de CN ;
R¹, R², R³, R⁴, R⁵ et R⁶ sont indépendemment H ou un alkyle en C₁₋₃ ;
R² peut également être un méthylène ou un éthylidène ;
R⁶ peut également être un alcényle ou un alcynyle en C₂₋₄
n vaut 0 ou 1 et
--- représente une simple ou double liaison, 2 doubles liaisons étant présentes au maximum.

8. Utilisation du 5-cyclopentyl-3-méthylpent-4-énal, du 4-cyclopentylpentanal ou du 5-cyclopentylhexanal en tant que parfum.

9. Utilisation selon les revendications 7 et 8 en parfumerie fine et fonctionnelle.
